# EUROPÄISCHE PATENTSCHRIFT

(11) **EP 2 129 340 B1**
(45) Veröffentlichungstag und Bekanntmachung des Hinweises auf die Patenterteilung: **13.03.2013**
(21) Anmeldenummer: 08715935.6
(22) Anmeldetag: 21.02.2008
(51) Int. Cl.: A61F 2/68

(54) **PROTHESE MIT LADBAREM, ELEKTRISCHEM ENERGIESPEICHER**
PROSTHESIS WITH CHARGEABLE ELECTRIC ENERGY ACCUMULATOR
PROTHÈSE DOTÉE D'UN ACCUMULATEUR D'ÉNERGIE ÉLECTRIQUE RECHARGEABLE

(30) Priorität: 23.02.2007 DE 102007009356
(43) Veröffentlichungstag der Anmeldung: 09.12.2009
(73) Patentinhaber: Otto Bock Healthcare Products GmbH, 1070 Wien (AT)
(72) Erfinder: PUCHHAMMER, Gregor, A-1130 Wien (AT); EGGER, Hubert, A-1220 Wien (AT)
(74) Vertreter: Plöger, Jan Manfred
(86) Internationale Anmeldenummer: PCT/EP2008/001377
(87) Internationale Veröffentlichungsnummer: WO 2008/101703

(56) Entgegenhaltungen:
- EP-A- 1 738 795
- WO-A-2006/022645
- WO-A-2007/096268
- WO-A-2007/096269
- WO-A-2008/033337
- US-A1- 2003 040 806

## Beschreibung

Die Erfindung betrifft eine Exoprothese mit einem elektrischen Energiespeicher.

In modernen Exoprothesen sind elektrische Aktoren vorhanden, um beispielsweise Glieder der Prothese zu bewegen oder um passive Elemente, wie beispielsweise Dämpfungselemente, in ihren Eigenschaften zu beeinflussen. Die von diesen Aktoren benötigte elektrische Energie wird in Akkus gespeichert. Dieser Akku muss in regelmäßigen Abständen aufgeladen werden. Dazu kann der Akku beispielsweise austauschbar sein. Ist der in der Prothese vorhandene Akku erschöpft, so wird die Prothese abgenommen und der erschöpfte Akku wird durch einen geladenen Akku ersetzt.

Nachteilig an die dieser Lösung ist, dass der Akku in regelmäßigen Absterben ausgetauscht werden muss, was lästig ist. Gemäß einer weiteren Alternative wird der Akku dadurch wieder aufgeladen, dass er über ein Ladekabel mit einer externen Stromquelle verbunden wird. Es hat sich jedoch gezeigt, dass diese Art, den Akku zu laden, fehleranfällig ist.

Aus der EP 1 738 795 A ist eine Vorrichtung zum Übertragen von elektrischer Energie durch die menschliche Haut bekannt, die mit einem elektrischen Energiespeicher und Induktionsspulen ausgestattet ist, wobei der Energiespeicher mittels elektrisch verbundener Energiespeicher-Ladeschaltung durch den von Induktionsspulen induzierten elektrischen Strom aufgeladen wird. Diese Vorrichtung dient der Versorgung von Endoprothesen, die sich hinsichtlich der Stromversorgung grundlegend von Exoprothesen unterscheiden. So stellt jedes Durchstoßen der menschlichen Haut eine potenzielle Keimquelle dar, was bei Exoprothesen nicht der Fall ist.

Aus der WO 2006/022645 A ist ein Verfahren zum Charakterisieren einer Batterie bekannt, die in einer Endoprothese vorgesehen ist. Wie bereits oben erwähnt, unterscheidet sich die Stromversorgung von Endoprothesen grundlegend in der Stromversorgung von Exoprothesen, da bei Exoprothesen beispielsweise keine Infektionsgefahr besteht.

Aus der US 2003/0040896 A1 ist eine weitere Endoprothese bekannt, die von außen drahtlos mit Strom versorgt werden kann.

Der Erfindung liegt die Aufgabe zugrunde, eine Exoprothese vorzuschlagen, bei der das Laden des Akkus mit einer verringerten Fehlerwahrscheinlichkeit möglich ist.

Die Erfindung löst das Problem durch eine gattungsgemäße Prothese, die eine Induktionsspule, eine mit der Induktionsspule und dem Energiespeicher elektrisch verbundene Ladeschaltung zum Laden des Energiespeichers aufgrund von einem in der Induktionsspule induzierten elektrischen Stroms und eine mit der Induktionsspule elektrisch verbundene Lastmodulationsschaltung aufweist, die mit der Induktionsspule Teil eines gemeinsamen Schaltkreises ist und die ausgebildet ist zum Einprägen einer Impedanzmodulation in den Schaltkreis.

Bei der Analyse der Fehler, die beim Laden von Akkus in Prothesen mittels Ladekabel auftreten, hat sich herausgestellt, dass es häufig zu Kontaktschwierigkeiten im Bereich des Steckers oder Buchse für den Stecker kommt. Der Grund hierfür ist, dass Patienten, die beispielsweise eine Armprothese tragen, den Stecker naturgemäß nur mit einer Hand in die Buchse in der Prothese einstecken können. Bei diesem Einsteckvorgang kann sich die Prothese bewegen. Um dieser Bewegung der Prothese zu begegnen, wird der Stecker, mit dem das Ladekabel an die Prothese angeschlossen wird, von dem Patienten in einem Ruck eingeführt, was die Buchse mechanisch stark belastet. Es kommt zudem durch Kontakt der Buchse mit Wasser oder Schmutz zu einem erhöhten Verschleiß von Stecker und Buchse, was durch die Patienten erneut durch ein Einführen des Steckers mit größerer Kraft kompensiert wird. Durch so hervorgerufene Kräfte kommt es leicht zu Kontaktproblemen zwischen der Buchse und deren Anschlüssen. Als ein weiterer Grund für Kontaktprobleme hat sich eine Verschmutzung des Steckers durch ein Konglomerat beispielsweise aus Schweiß mit Kleiderfasern, Kosmetikschaumabrieb, Staub und Ähnlichem herausgestellt. Um so verursachte Kontaktprobleme zu beheben, führen Patienten den Stecker mit einer größeren Kraft ein, was Stecker und Buchse belastet.

Dadurch, dass erfindungsgemäß vorgesehen ist, dass der Akku der Exoprothese über die Induktionsspule aufgeladen werden kann, wird eine mechanische Belastung der beteiligten Komponenten wie Stecker, Buchse und zugehörige Anschlüsse deutlich reduziert. Hieraus resultiert vorteilhafterweise eine deutlich verlängerte Lebensdauer der Prothese.

Vorteilhaft ist zudem, dass die Außenhülle der Prothese einheitlich mit einer hautfarbenen Schutzhülle versehen sein kann, die nicht durch eine Buchse unterbrochen werden muss. Das verbessert den ästhetischen Eindruck der Prothese und erhöht den Tragekomfort bei dem Patienten. Es ist ein weiterer Vorteil, dass durch das Vorsehen der Induktionsspule eine ununterbrochene und damit spritzwasserfeste Umhüllung der Prothese ermöglicht wird. Die Prothese wird so robuster und benutzerfreundlicher.

Vorteilhaft ist zudem, dass die Exoprothese aufgrund des Vorhandenseins der Induktionsspule zum Laden in eine geeignet geformte Ladevorrichtung gelegt werden kann. Der Vorgang des Anschließens eines Steckers oder einer sonstigen externen Komponente kann dann vollständig entfallen und reduziert das Gefühl bei dem Patienten, dass die Prothese eine Maschine ist. Hierdurch wird die Akzeptanz der Prothese beim Patienten erhöht.

Bei der erfindungsgemäßen Prothese handelt es sich insbesondere um eine Exoprothese, die von außen zugänglich uns vom Körper des Patienten lösbar ist.

Erfindungsgemäß ist die Induktionsspule elektrisch mit einer Lastmodulationsschaltung verbunden, die mit der Induktionsspule Teil eines gemeinsamen Schaltkreises ist und die ausgebildet ist zum Einprägen einer Impedanzmodulation in den Schaltkreis. Vorteilhaft hieran ist, dass mittels der Impedanzmodulation Informationen von der Prothese an eine mit der Induktionsspule zusammenwirkende Induktionsspule gesendet werden können. Wenn die Prothese beispielsweise einen Mikroprozessor umfasst, der Betriebszyklen oder Fehlermeldungen von elektrischen Komponenten der Prothese erfasst, so können diese Betriebsdaten über die Impedanzmodulation übermittelt werden.

In einer bevorzugten Ausführungsform umfasst die Exoprothese eine geschlossene wasserdichte Außenhülle. Unter einer geschlossenen wasserdichten Außenhülle ist insbesondere zu verstehen, dass dann, wenn die Prothese am Körper des Patienten befestigt ist, kein Spritzwasser an andere Komponenten der Prothese gelangen kann. Dadurch sind im Inneren der Außenhülle angeordnete Komponenten, wie beispielsweise Motoren und Mikroprozessoren, gegen Kontakt mit Spritzwasser geschützt. Das erhöht die Betriebssicherheit der Prothese. Eine solche Prothese ist zudem nach außen vollständig elektrisch isoliert, was mikroelektronische Bauteile vor Schäden durch elektrische Aufladungen schützt. Von außen zugängliche elektrische Kontakte können nämlich solche elektrostatischen Ladungen, die beispielsweise beim Ausziehen eines Pullovers durch den Patienten entstehen können, zu den mikroelektrischen Bauteilen leiten.

Um die Beweglichkeit der Prothese nicht zu beeinträchtigen, ist die Induktionsspule bevorzugt flexibel ausgebildet.

Bevorzugt wird die Exoprothese mit einer von der Prothese entfernbaren Ladespule zum Zusammenwirken mit der Induktionsspule versehen, so dass sich ein Prothesensystem ergibt. Die Induktionsspule ist dabei auf eine Eigenfrequenz der Ladespule abgestimmt, so dass dann, wenn die Ladespule mit einem Wechselstrom beaufschlagt und in die Nähe der Induktionsspule gebracht wird, in der Induktionsspule ein elektrischer Strom induziert wird, der den Energiespeicher elektrisch auflädt. Die Ladespule ist über mindestens ein Ladekabel mit einer Stromquelle verbunden, um die Ladespule mit einem Laststrom zu versorgen, wobei die Stromquelle ausgebildet ist zum Aufprägen von Steuersignalen für die Prothesen auf den Ladestrom. Das Aufprägen kann beispielsweise dadurch erfolgen, dass die Amplitude des Ladestroms variiert wird. Alternativ kann auf den Ladestrom ein höherfrequenter elektrischer Strom addiert werden. Es ist zudem möglich, den Ladestrom frequenz-zu modulieren.

Die Stromquelle kann beispielsweise ausgebildet sein, um solche Steuersignale an die Prothese zu übermitteln, die Befehle kodiert, um Betriebsparameter von der Prothese auszulesen.

Eine besonders sichere Verbindung zwischen der Induktionsspule und der Ladespule wird erhalten, wenn die Ladespule zum formschlüssigen Verbinden mit der Prothese ausgebildet ist. Das kann dadurch geschehen, dass die Ladespule selbst eine entsprechende geometrische Form hat. Alternativ kann die Ladespule von einer Hülle umgeben sein, die zum formschlüssigen Verbinden mit der Prothese ausgebildet ist.

Besonders vorteilhaft ist es, wenn die Prothese eine Handprothese ist, die mindestens einen Finger umfasst, wobei die Ladespule zum ringartigen Umgreifen des Fingers ausgebildet ist. In diesem Fall kann die Ladespule einfach wie ein Schmuckring über einen der Finger gezogen werden und wird so sicher an der Prothese fixiert. Alternativ kann vorgesehen sein, dass die Ladespule an einem armringförmigen Halter befestigt ist, der beispielsweise durch eine Bewegung radial auf ein Handgelenk der Prothese zu mit der Prothese reversibel verbindbar ist.

Ein besonders einfach zu bedienendes Prothesensystem ergibt sich, wenn die Ladespule einen Magneten umfasst, der zum Zusammenwirken mit einem ferromagnetischen Bauteil der Induktionsspule, insbesondere mit einem Kern der Induktionsspule, ausgebildet ist, so dass die Ladespule aufgrund einer magnetischen Anziehungskraft an der Prothese reversibel befestigbar ist. In diesem Fall muss die Ladespule lediglich in die Nähe der Induktionsspule gebracht werden. Sodann zieht der Magnet den ferromagnetischen Kern der Induktionsspule an und die Induktionsspule ist kraftschlüssig mit der Ladespule verbunden. Dabei beeinflusst der Magnet das Übertragungsverhalten von Energie und Daten nicht.

Besonders bevorzugt ist der Magnet so ausgebildet, dass er sich unter mechanischer Last von der Prothese löst, bevor der Magnet oder das Ladekabel beschädigt werden. Fällt die Prothese beispielsweise herunter, während sie mit der Ladespule verbunden ist, so folgt daraus kein Schaden für Prothese oder Ladespule. Bei einem Stecker-Buchse-System nach dem Stand der Technik wären hingegen Schäden zu befürchten.

Besonders bevorzugt ist die Ladespule wasserdicht eingekapselt. Dadurch wird es möglich, die Prothese auch während einer spritzwassergefährdeten Arbeit aufzuladen.

Es kann vorgesehen sein, dass der Magnet ein mit der Stromquelle verbundener Elektromagnet ist, wobei die Stromquelle ausgebildet ist, um den Magneten zu deaktivieren, wenn der Energiespeicher gefüllt ist. In diesem Fall fungiert der Magnet als Haltemagnet, der dann abgeschaltet wird, wenn er nicht benötigt wird, um die Induktionsspule und die Ladespule räumlich dicht beieinander zu halten. Im nicht-aktivierten Zustand zieht der Elektromagnet vorteilhafterweise keine ferromagnetischen Gegenstände an. Für den Benutzer ist somit sehr leicht feststellbar, ob der Ladevorgang beendet ist oder nicht. Der Ladezustand des Energiespeichers wird bevorzugt über eine LED-Anzeige dargestellt.

Um die Induktionsspule besonders einfach beispielsweise über einen Finger der Prothese schieben zu können, ist die Induktionsspule bevorzugt flexibel ausgebildet.

Bevorzugt ist die Stromquelle so ausgebildet, dass sich dann, wenn sich die Induktionsspule nicht in unmittelbarer Nähe befindet, in vorgegebenen zeitlichen Abständen in der Größenordnung von Sekunden ein im Vergleich zum Ladebetrieb stark reduziert magnetisches Wechselfeld erzeugt. Wird dieses Wechselfeld von der Induktionsspule erfasst, so verändert die Lastmodulationsschaltung die Impedanz des Schaltkreises auf eine vorgegebene Weise. Die Rückkopplung dieser veränderten Impedanz wird von der Stromquelle bzw. einer mit ihr verbundenen Steuerung erfasst und auf Erzeugung eines Lade-Wechselfelds angeschaltet. Die beteiligten Komponenten sind so ausgebildet, dass das Lade-Wechselfeld erst aufgebaut wird, wenn sich Induktionsspule und Ladespule in unmittelbarer Nähe zueinander befinden. Dadurch werden als Sicherheitsmaßnahme Schäden durch das Lade-Wechselfeld an Menschen, die beispielsweise einen Herzschrittmacher tragen, vermieden.

Im Folgenden wird ein Ausführungsbeispiel der Erfindung anhand der beigefügten Zeichnungen näher erläutert. Dabei zeigt
- Figur 1: ein erfindungsgemäßes Prothesensystem mit einer erfindungsgemäßen Prothese,
- Figur 2: ein alternatives Prothesensystem, bei dem die Ladespule an einem Finger befestigt ist,
- Figur 3: eine Ladespule und eine Induktionsspule für eine erfindungsgemäße Prothese,
- Figur 4: einen Finger einer erfindungsgemäßen Prothese mit einer einen Kern aufweisenden Induktionsspule und
- Figur 5: einen Finger einer erfindungsgemäßen Prothese mit einer kernlosen Induktionsspule.

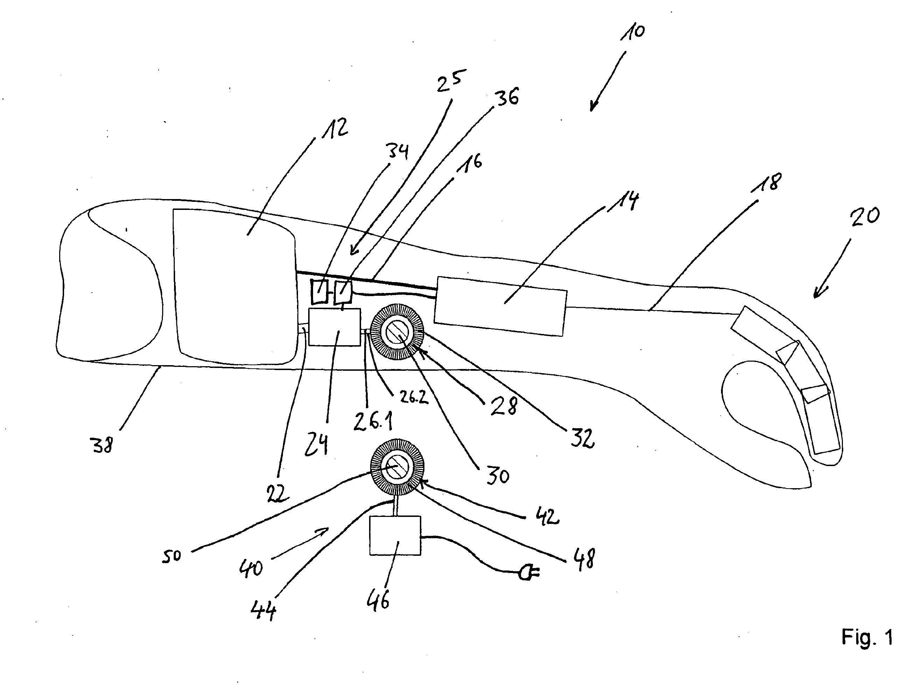

Figur 1 zeigt eine Prothese 10 in Form einer Unterarmprothese, die einen Energiespeicher in Form eines Akkus 12 und einen Elektromotor 14 umfasst, der mittels eines Kabels 16 mit dem Akku 12 verbunden ist. Der Elektromotor 14 kann über ein Zugseil 18 ein Fingergelenk 20 betätigen. Alternativ ist der Elektromotor 14 mittels eines Gelenks mit dem Fingergelenk verbunden.

Der Akku 12 ist über eine elektrische Leitung 22 mit einer Ladeschaltung 24 verbunden, die ihrerseits über einen elektrischen Draht 26 mit einer Induktionsspule 28 in elektrischem Kontakt steht. Die Induktionsspule 28 besitzt einen ferromagnetischen Kern 30, der inmitten einer Wicklung 32 angeordnet ist.

Wenn die Induktionsspule 28 in ein magnetisches Wechselfeld gelangt, so wird in der Wicklung 32 eine Spannung induziert, die durch die Ladeschaltung 24 gleichgerichtet und auf eine vorgegebene Spannung gebracht wird. Der so gleichgerichtete elektrische Strom wird an den Akku 12 angelegt, so dass der Akku 12 geladen wird. Der Kern 30 dient der Erhöhung des elektrischen Kopplungsgrades der Induktionsspule 28.

Die Ladeschaltung 24 umfasst eine Lastmodulationsschaltung 25, die mit den Drähten 26.1, 26.2 und der Induktionsspule 28 einen Schaltkreis bildet. Die Lastmodulationsschaltung ist ausgebildet, um die Impedanz dieses Schaltkreises zu modulieren. Auf diese Weise können Daten aus einem digitalen Datenspeicher 34 eines Mikrocontrollers 36, die Teil der Lastmodulationsschaltung 25 sind, nach außen übertragen werden. Beispielsweise kann mit der Lastmodulationsschaltung 25 ein Ladezustand des Akkus 12 codiert und nach außen übertragen werden.

Die oben beschriebenen elektrischen Komponenten der Prothese 10 sind von einer geschlossenen wasserdichten Außenhülle 38 aus PVC oder Silikon umgeben, die die elektrischen Komponenten vor Spritzwasser schützt. Für Prothesen, bei denen die Wasserdichtigkeit eine besondere Rolle spielt, kann vorgesehen sein, dass die Außenhülle 38 vollständig geschlossen ist, so dass auch bei einem Eintauchen der Prothese 10 in Wasser keine elektrische Komponente nass wird.

Die Prothese wirkt mit einem Ladegerät 40 zusammen, das eine Ladespule 42 umfasst, die über ein Ladekabel 44 mit einer Stromquelle 46 elektrisch verbunden ist.

Die Ladespule 42 wird im Betrieb von einem Wechselstrom durchflossen, der in der Ladespule 42 ein magnetisches Wechselfeld erzeugt. Wenn die Ladespule 42 in hinreichende Nähe zu der Induktionsspule 28 gebracht wird, wird so in der Induktionsspule 28 wie oben beschrieben ein Wechselstrom induziert. Die Ladespule 42 wird dazu mit einem Wechselstrom betrieben, der die Resonanzfrequenz der Induktionsspule 28 trifft. Die Ladespule 42 und die Induktionsspule 28 werden auf Resonanz nachgeregelt. Ändert sich die Kopplung zwischen beiden Spulen, wird innerhalb eines vorbestimmten Arbeitsbereichs so nachgeregelt, dass Energie- und Datenübertragung stets optional sind.

Zum Befestigen der Ladespule 42 in der Nähe der Induktionsspule 28 ist inmitten einer Wicklung 48 der Ladespule 42 ein Magnet 50 angeordnet. Wenn die Ladespule 42 in Nähe der Induktionsspule 28 gerät, zieht der Magnet 50 den Kern 30 an und fixiert so Induktionsspule 28 und Ladespule 42 aneinander. Die Stärke des Magneten 50 ist dabei so gewählt, dass die Verbindung zwischen dem Magnet 50 und dem Kern 30 schwächer ist als die mechanische Verbindung zwischen dem Ladekabel 44 und der Ladespule 42, so dass sie sich bei einem Zug an dem Ladekabel 44 löst, bevor ein Schaden eintreten kann.

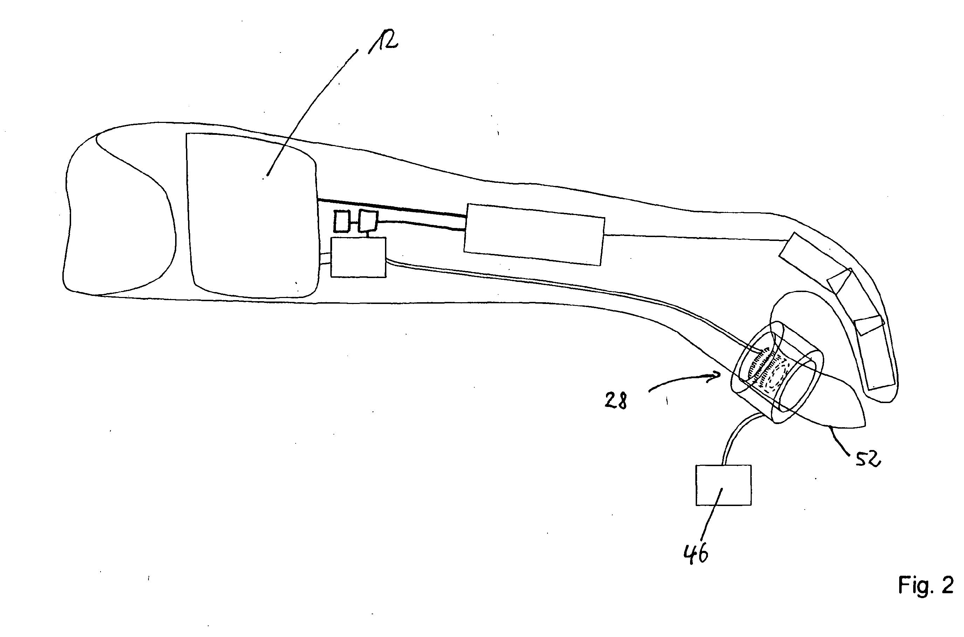

Figur 2 zeigt ein alternatives Prothesensystem, das sich von dem in Figur 1 gezeigten Prothesensystem dahingehend unterscheidet, dass die Induktionsspule 28 in einem Finger 52 in Form eines Daumens angeordnet ist. Die Ladespule 42 ist so ausgebildet, dass sie über den Finger 52 geschoben und diesen ringartig umgreifen kann. Auf diese Weise wird eine besonders einfache und bedienungssichere Übertragung von elektrischer Energie an den Akku 12 gewährleistet.

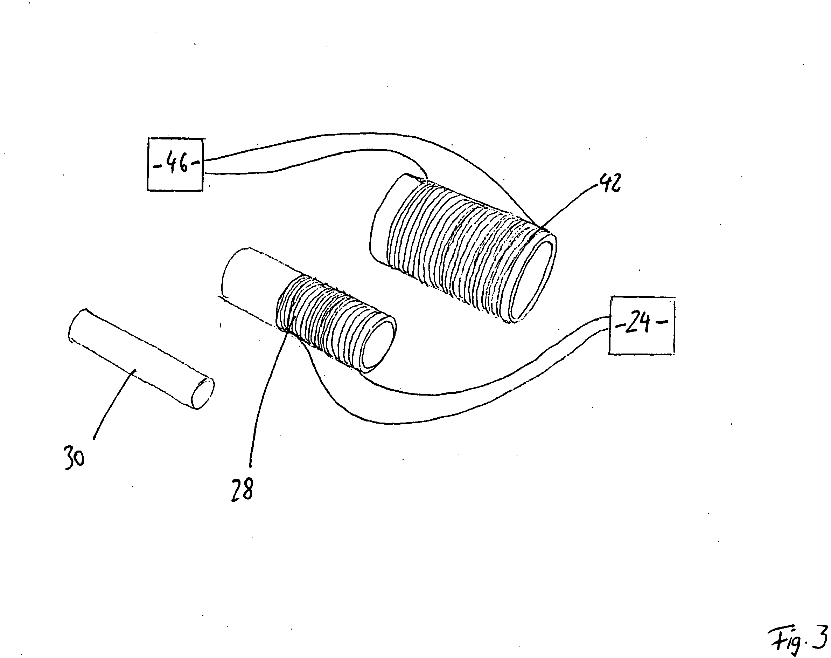

Figur 3 zeigt die Ladespule 42, die Induktionsspule 28 und den Kern 30 für die Ladespule 28.

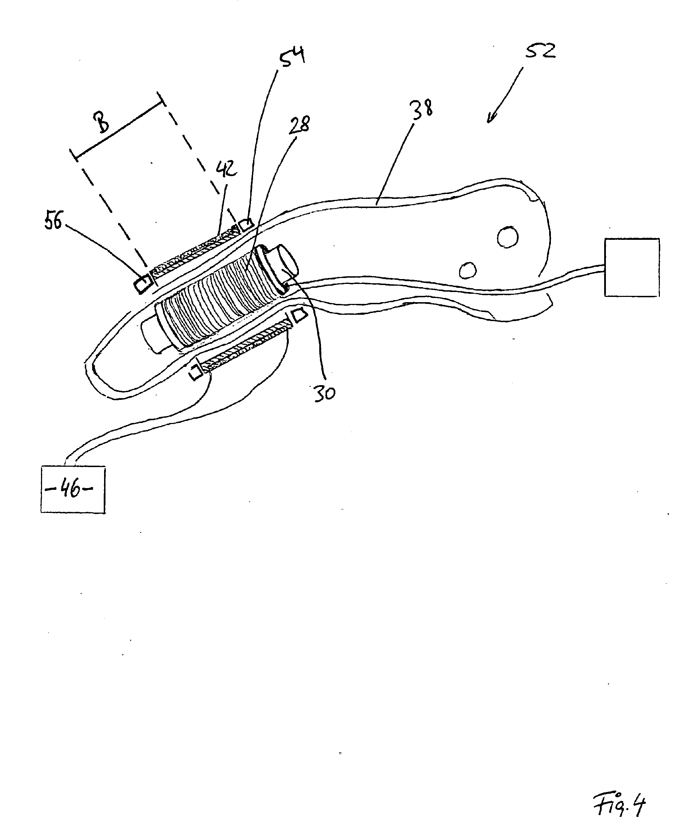

Figur 4 zeigt eine schematische Ansicht eines Fingers 52, der kein Daumen ist, in dem eine einen Kern 30 aufweisende Induktionsspule 28 im Bereich der vorderen beiden Fingerglieder angeordnet ist. Die Ladespule 72 ist formschlüssig mit dem Finger 52 verbunden, indem sie auf den Finger 52 aufgesteckt worden ist. Eine axiale Fixierung der Codespule 42 kann dadurch erreicht werden, dass an beiden Enden der Codespule 42 jeweils ein Magnet 54, 56 angebracht ist, so dass das dabei aufgebaute Magnetfeld sich zum Kern 30 ausrichten will. Die Ladespule 42 weist eine Breite B auf, die einem Abstand zwischen einem ersten Fingergelenk und einem zweiten Fingergelenk des Fingers 52 entspricht. Die Breite der Induktionsspule 28 ist um einen kleinen Betrag, beispielsweise um 10 bis 20 %, größer als die Breite B der Ladespule 42.

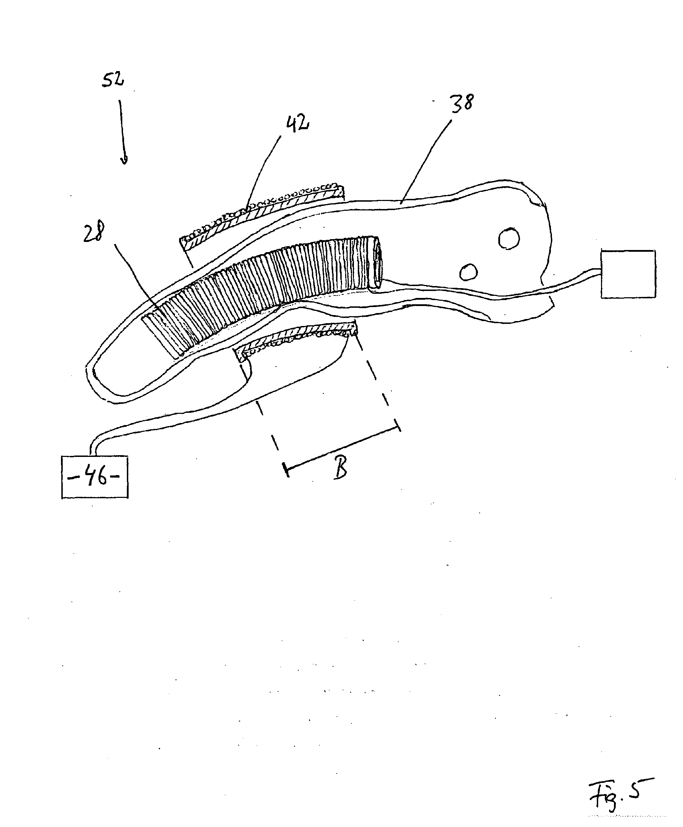

Figur 5 zeigt eine kernlose, flexible Induktionsspule 28, deren Breite mehr als 20 % größer ist als die Breite B der Ladespule 42. Durch die größere Breite kann der fehlende Kern 30 zumindest teilweise kompensiert werden.

### Bezugszeichenliste

- 10: Prothese
- 12: Akku
- 14: Elektromotor
- 16: Kabel
- 18: Zugseil

- 20: Fingergelenk
- 22: Leitung
- 24: Ladeschaltung
- 25: Lastmodulationsschaltung
- 26.1, 26.2: Draht

- 28: Induktionsspule
- 30: Kern
- 32: Wicklung
- 34: digitaler Datenspeicher
- 36: Mikrocontroller

- 38: Außenhülle
- 40: Ladegerät
- 42: Ladespule
- 44: Ladekabel
- 46: Stromquelle

- 48: Wicklung
- 50: Magnet
- 52: Finger
- 54: Magnet
- 56: Magnet
- B: Breite

## Patentansprüche

1. **Exoprothese** mit einem elektrischen Energiespeicher (12),
(a) einer Induktionsspule (28),
(b) einer mit der Induktionsspule (28) und dem Energiespeicher (12) elektrisch verbundenen Ladeschaltung (24) zum Laden des Energiespeichers (12) aufgrund von einem in der Induktionsspule (28) induzierten elektrischen Strom und
(c) einer mit der Induktionsspule (28) elektrisch verbundenen Lastmodutationsschaltung (25), die mit der Induktionsspule (28) Teil eines gemeinsamen Schaltkreises ist und die ausgebildet ist zum Einprägen einer Impedanzmodulation in den Schaltkreis.

2. Exoprothese nach einem der vorstehenden Ansprüche, **dadurch gekennzeichnet, dass** sie eine geschlossene wasserdichte Außenhülle (38) besitzt.

3. Exoprothese nach einem der Ansprüche, **dadurch gekennzeichnet, dass** die Induktionsspule flexibel ist.

4. Prothesensystem mit einer Exoprothese (10) nach einem der vorstehenden Ansprüche, **gekennzeichnet durch** eine von der Exopothese (10) entfernbare Ladespule (44) zum Zusammenwirken mit der Induktionsspule (28).

5. Prothesensystem nach Anspruch 4, **dadurch gekennzeichnet, dass** die Ladespule (42) über mindestens ein Ladekabel (44) mit einer Stromquelle (46) in Verbindung steht, um die Ladespule (42) mit einem Ladestrom zu versorgen, und die Stromquelle (46) ausgebildet ist zum Aufprägen von Steuersignalen für die Exoprothese (10) auf den Ladestrom.

6. Prothesensystem nach einem der Ansprüche 4 oder 5, **dadurch gekennzeichnet, dass** die Ladespule (42) ausgebildet ist zum formschlüssigen Verbinden mit der Exoprothese (10).

7. Prothesensystem nach einem der Ansprüche 4 bis 6, **dadurch gekennzeichnet, dass** die Exoprothese (10) eine Handprothese ist, die mindestens einen Finger (52) umfasst, und dass die Ladespule (42) zum ringartigen Umgreifen des Fingers (52) ausgebildet ist.

8. Prothesensystem nach einem der Ansprüche 4 bis 7, **dadurch gekennzeichnet, dass** die Ladespule (42) einen Magneten (50) umfasst zum Zusammenwirken mit einem ferromagnetischen Bauelement (30) der Induktionsspule (28), insbesondere mit einem Kern (30) der Induktionsspule (28), so dass die Ladespule (42) aufgrund einer magnetischen Anziehungskraft an der Exoprothese (10) reversibel befestigbar ist.

9. Prothesensystem nach Anspruch 8, **dadurch gekennzeichnet, dass** der Magnet (50) so ausgebildet ist, dass sich der Magnet (50) unter mechanischer Last von der Exoprothese (10) löst, bevor der Magnet (50) oder das mindestens eine Ladekabel (44) beschädigt wird.

10. Prothesensystem nach einem der Ansprüche 4 bis 9, **dadurch gekennzeichnet, dass** die Ladespule (42) wasserdicht gekapselt ist.

11. Prothesensystem nach einem der Ansprüche 8 bis 10, **dadurch gekennzeichnet, dass**
- der Magnet (50) ein mit der Stromquelle (46) verbundener Elektromagnet ist und
- die Stromquelle ausgebildet ist, um den Magneten (50) zu deaktivieren, wenn der Energiespeicher (12) gefüllt ist.

12. Prothesensystem nach einem der Ansprüche 4 bis 11, **dadurch gekennzeichnet, dass** die Induktionsspule flexibel ist.

## Claims

1. An exoprothesis with an electric energy storage device (12),
(a) an induction coil (28),
(b) a charging circuit (24) which is electrically connected to the induction coil (28) and the energy storage device (12) for charging the energy storage device (12) on the basis of an electric current induced in the induction coil (28) and
(c) a load modulation circuit (25) which is electrically connected to the induction coil (25), which, along with the induction coil (28), is part of a circuit and is designed to cause an impedance modulation in the circuit.

2. The exoprothesis according to one of the above claims, **characterized by** the fact that it comprises a closed, waterproof outer shell (38).

3. The exoprothesis according to one of the above claims, **characterized by** the fact that the induction coil is flexible.

4. A prothesis system with an exoprothesis (10) according to one of the above claims, **characterized by** a charging coil (44), which can be detached from the exoprothesis, to interact with the induction coil (28).

5. The prothesis system according to claim 4, **characterized by** the fact that the charging coil (42) is connected to a power source (46) by at least one charging cable (44), in order to supply the charging coil (42) with a charging current, and that the power source (46) is designed to create control signals for the exoprothesis (10) in the charging current.

6. The prothesis system according to one of the claims 4 or 5, **characterized by** the fact that the charging coil (42) is designed to positively connect with the exoprothesis (10).

7. The prothesis system according to one of the claims 4 to 6, **characterized by** the fact that the exoprothesis (10) is a hand prothesis which comprises at least one finger (52), and that the charging coil (42) is designed to encompass the finger (52) like a ring.

8. The prothesis system according to one of the claims 4 to 7, **characterized by** the fact that the charging coil (42) comprises a magnet (50) to interact with a ferromagnetic building element (30) of the induction coil (28), in particular with a core (30) of the induction coil (28), so that the charging coil (42) can be reversibly attached to the exoprothesis (10), due to a magnetic force of attraction.

9. The prothesis system according to claim 8, **characterized by** the fact that the magnet (50) is designed in such a way that the magnet (50) releases itself under the mechanical load of the exoprothesis (10) before the magnet (50) or at least one charging cable (44) is damaged.

10. The prothesis system according to one of the claims 4 to 9, **characterized by** the fact that the charging coil (42) is enclosed so that it is waterproof.

11. The prothesis system according to one of the claims 8 to 10, **characterized by** the fact that
- the magnet (50) is an electro-magnet that is connected to the power source (46) and
- the power source is designed to deactivate the magnet (50) when the energy storage device is full.

12. The prothesis system according to one of the claims 4 to 11, **characterized by** the fact the induction coil is flexible.

## Revendications

1. Exoprothèse comprenant un accumulateur d'énergie électrique (12),
(a) une bobine d'induction (28),
(b) un circuit de charge (24) connecté électriquement à la bobine d'induction (28) et à l'accumulateur d'énergie (12) pour charger l'accumulateur d'énergie (12) au moyen d'un courant électrique induit dans la bobine d'induction (28), et
(c) un circuit de modulation de charge (25) connecté électriquement à la bobine d'induction (28), qui fait partie avec la bobine d'induction (28) d'un circuit de commutation commun et qui est réalisé pour imposer une modulation d'impédance dans le circuit de commutation.

2. Exoprothèse selon l'une des revendications précédentes, **caractérisée en ce qu'**elle possède une enveloppe extérieure (38) fermée étanche à l'eau.

3. Exoprothèse selon l'une des revendications précédentes, **caractérisée en ce que** la bobine d'induction est flexible.

4. Système de prothèse comprenant une exoprothèse (10) selon l'une des revendications précédentes, **caractérisé par** une bobine de charge (44) susceptible d'être enlevée de l'exoprothèse (10), pour coopérer avec la bobine d'induction (28).

5. Système de prothèse selon la revendication 4, **caractérisé en ce que** la bobine de charge (42) est connectée à une source de courant (46) via au moins un câble de charge (44), afin d'alimenter la bobine de charge (42) avec un courant de charge, et la source de courant (46) est réalisée pour superposer des signaux de commande pour l'exoprothèse (10) sur le courant de charge.

6. Système de prothèse selon l'une des revendications 4 ou 5, **caractérisé en ce que** la bobine de charge (42) est réalisée pour être reliée avec l'exoprothèse (10) en coopération de formes.

7. Système de prothèse selon l'une des revendications 4 à 6, **caractérisé en ce que** l'exoprothèse (10) est une prothèse de la main, qui comprend au moins un doigt (52), et **en ce que** la bobine de charge (42) est réalisée de façon à entourer le doigt (52) à la manière d'une bague.

8. Système de prothèse selon l'une des revendications 4 à 7, **caractérisé en ce que** la bobine de charge (42) comprend un aimant (50) pour coopérer avec un élément structurel ferromagnétique (30) de la bobine d'induction (28), en particulier avec un noyau (30) de la bobine d'induction (28), de sorte que la bobine de charge (42) est susceptible d'être fixée de manière réversible sur l'exoprothèse (10) grâce à une force d'attraction magnétique.

9. Système de prothèse selon la revendication 8, **caractérisé en ce que** l'aimant (50) est réalisé de telle manière que l'aimant (50) se détache de l'exoprothèse (10) sous une charge mécanique avant que l'aimant (50) ou ledit au moins un câble de charge (44) soit endommagé.

10. Système de prothèse selon l'une des revendications 4 à 9, **caractérisé en ce que** la bobine de charge (42) est encapsulée de manière étanche à l'eau.

11. Système de prothèse selon l'une des revendications 8 à 10, **caractérisé en ce que**
- l'aimant (50) est un électroaimant connecté à la source de courant (46), et
- la source de courant est réalisée pour désactiver l'aimant (50) quand l'accumulateur d'énergie (12) est rempli.

12. Système de prothèse selon l'une des revendications 4 à 11, **caractérisé en ce que** la bobine d'induction est flexible.
